Europäisches Patentamt

**European Patent Office**

**Office européen des brevets**

(19)

(11) Numéro de publication: **0 107 590**
**A1**

# (12) DEMANDE DE BREVET EUROPEEN

(21) Numéro de dépôt: **83402035.6**

(22) Date de dépôt: **20.10.83**

(51) Int. Cl.³: **A 61 M 16/00**

(30) Priorité: **22.10.82 FR 8217697**

(43) Date de publication de la demande: **02.05.84**
**Bulletin 84/18**

(84) Etats contractants désignés: **AT BE CH DE FR GB IT LI**
**LU NL SE**

(71) Demandeur: **Sarl dite: ROBERT ET CARRIERE-**
**DRAEGER, 27 rue de la Glacière, F-75013 Paris (FR)**
Demandeur: **S.A. dite LAMA, 19 rue du Gord,**
**F-91580 Etrechy (FR)**

(72) Inventeur: **Le Masson, Yves, 6 rue Saint Martin,**
**F-91150 Etampes (FR)**

(74) Mandataire: **Pinguet, André, CAPRI 28 bis, avenue**
**Mozart, F-75016 Paris (FR)**

(54) **Ranimateur hyperbaré à commande pneumatique.**

(57) L'appareil est destiné à réanimer des patients dans une chambre en surpression dans laquelle on cherche à éviter tout matériel électrique. Les logiques pneumatiques usuelles ne fonctionnent pas correctement dans une chambre en surpression. Selon l'invention, une cellule pneumatique logique (11) est alimentée par un organe (12) fournissant une pression de valeur absolue constante et l'échappement de la cellule est renvoyé à l'extérieur de la chambre hyperbare (10), le conduit d'échappement (13) comportant une résistance de charge (14), et une dérivation (16), branchée entre l'échappement de la cellule (11) et la résistance, et connectée à un organe (18) de commutation sensible à la pression, pour commander l'insufflation du patient, qui provient d'une source de gaz respirable dont la pression relative est proportionnelle à la pression régnant dans le caisson.

- 1 -

Ranimateur hyperbare à commande pneumatique

La présente invention concerne les matériels de ranimation tels que les respirateurs artificiels et, plus particulièrement, les matériels de ce type fonctionnant dans des espaces clos sous pression désignés caissons ou chambres hyperbares. L'invention a pour objet un dispositif pour commander le débit volumétrique, la fréquence et le rapport de l'inspiration à l'expiration dans des conditions de surpression, avec une totale fiabilité.

Les chambres hyperbares sont utilisées pour ranimer des patients dont l'état nécessite des conditions particulières telles que surpression, haute teneur en oxygène, etc, qui recommandent fortement d'éviter les dispositifs électriques ou électroniques, quelles que soient les précautions antidéflagrantes. On préfère donc utiliser des dispositifs entièrement pneumatiques. Mais, dans les conditions de pression des chambres hyperbares, les logiques pneumatiques dérivent de façon incontrôlée et il n'est plus possible d'assurer la stabilité du rapport I/E (de la durée d'inspiration à la durée d'expiration), de la fréquence et du débit volumétrique pendant la période d'inspiration. Le débit volumétrique doit être en effet indépendant de la pression qui peut selon les applications, être de 1 à quelques bar.

La présente invention a pour objet un ranimateur pour chambre hyperbare, à commande pneumatique, remarquable notamment en ce qu'il comporte une cellule pneumatique logique, un organe d'alimentation de gaz à pression de valeur absolue constante pour la commande de ladite cellule, dont l'échappement est renvoyé à l'extérieur de la chambre hyperbare, le conduit d'échappement comportant une résistance de charge, et une dérivation, branchée entre l'échappement de la cellule et la résistance, et connectée à un organe de commutation sensible à la pression, pour commander le passage du gaz respirable.

Il existe différents types de cellules pneumatiques logiques pour assurer la régulation de la fréquence des insufflations. Dans le ranimateur selon l'invention, la cellule peut être d'un quelconque type connu.

L'organe de commutation peut être avantageusement un distributeur pneumatique à tiroir ou à piston, dont une première face est exposée à la pression régnant dans la dérivation de l'échappement, la seconde face symétrique du piston étant exposée à la pression régnant à l'extérieur de la chambre hyperbare, c'est-à-dire normalement la pression atmosphérique, mais qui peut aussi être une autre pression de référence.

Quand un signal est délivré à la sortie de la cellule, il règne dans la dérivation une pression supérieure à la pression atmosphérique, du fait de la résistance de charge sur le conduit d'échappement. Cette pression est appliquée sur la première face du piston qui se déplace et commande le fonctionnement du ranimateur. En l'absence d'un signal de sortie à l'échappement de la cellule, le piston occupe une position de repos.

Le distributeur relie une source de gaz respirable sous une pression proportionnelle à la pression régnant dans la chambre à une conduite de distribution du gaz au malade, laquelle conduite est avantageusement munie d'un orifice réglable.

D'autres caractéristiques de l'invention apparaîtront au cours de la description qui va suivre, donnée à titre d'exemple non limitatif en regard des dessins ci-joints, et qui fera bien comprendre comment l'invention peut être réalisée.

0107590

- 3 -

Sur les dessins,
- la figure 1 représente un schéma de principe du respirateur selon l'invention ; et,
- la figure 2 représente un schéma plus détaillé du respirateur de la figure 1.

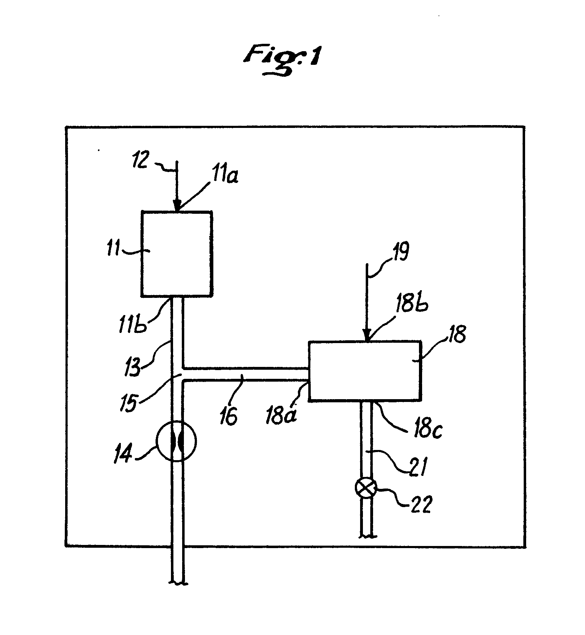

Sur la figure 1, le ranimateur installé dans la chambre
hyperbare 10 comprend une cellule logique pneumatique 11
déterminant la fréquence de la respiration, et le rapport
I/E du temps d'inspiration au temps d'expiration. Il existe
différents types de telles cellules et le choix n'est pas
critique pour la présente invention. La cellule 11 est
munie à son entrée 11a de moyens 12 d'alimentation en gaz
à une pression de valeur absolue constante, provenant de
l'intérieur ou de l'extérieur de la chambre 10, des moyens
étant par ailleurs prévus pour maintenir à l'intérieur de
la chambre la pression désirée, qui, selon les cas, peut
aller de 1 à 5 bar environ. La conduite d'échappement 13
à la sortie 11b de la cellule conduit hors de la chambre
10, à la pression atmosphérique dans les conditions générales. Sur la conduite 13, est montée une résistance 14,
tel qu'un étranglement. Entre la sortie 11b de la cellule
et la résistance 14 est branchée en 15 une dérivation 16
connectée à une entrée de commande 18a d'un distributeur
18. Ce distributeur 18 est relié par une entrée 18b à une
source 19 de gaz respirable sous une pression proportionnelle à la pression régnant dans la chambre. Le distributeur 18 est d'autre part relié au masque respiratoire
par une conduite 21 branchée à la sortie 18c. La conduite
21 est avantageusement munie d'un réglage 22. Les organes
et conduites d'expiration connus en eux-mêmes ne sont pas
représentés.

Le dispositif fonctionne de la façon suivante. Quand il
n'y a aucun signal à la sortie 11b de la cellule, aucune
pression, aucun débit, la pression extérieure à la cham-

- 4 -

bre (pression atmosphérique), règne dans les conduites 13 et 16. Quand un signal de pression est délivré à la sortie 11b de la cellule, il règne en amont de la résistance 14, et par conséquent dans la conduite 16 jusqu'au distributeur 18, une pression supérieure. Le fonctionnement du distributeur 18 est donc commandé par le niveau de pression présent à son entrée 18a.

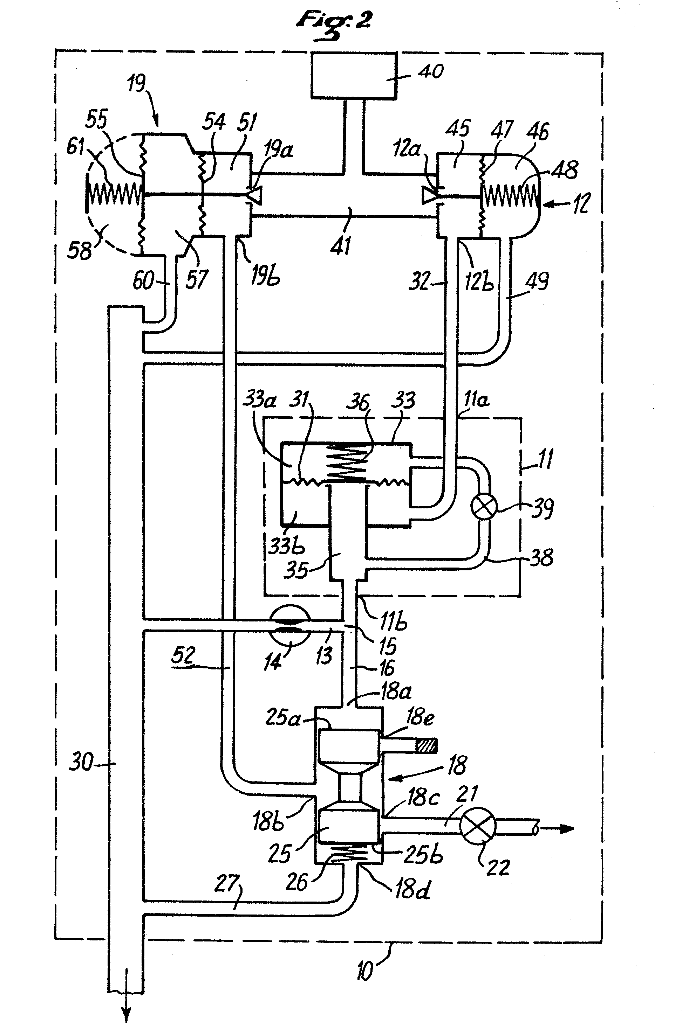

La figure 2 illustre à titre d'exemple nullement limitatif comment peuvent être réalisés le distributeur, la cellule logique 11 et les alimentations 12 en gaz à pression de valeur absolue constante et 19 en gaz respirable sous une pression proportionnelle à la pression régnant dans le caisson.

Le distributeur 18 est un distributeur à une entrée 18b et deux sorties 18c, 18e, dont l'une, inutile, est obturée. Il renferme un piston 25 dont la première face 25a est exposée à la pression de commande 18a. La seconde face 25b est sollicitée par un ressort 26 vers la position représentée, dans laquelle la sortie 18c est fermée. L'extrémité du distributeur 18, correspondant à la seconde face 25b du piston, est mise en communication avec la pression atmosphérique par une canalisation 27 connectée, d'un côté à un orifice de sortie 18d, et de l'autre à un collecteur 30 en communication avec l'extérieur de la chambre 10, c'est-à-dire avec la pression atmosphérique. Ce collecteur peut recevoir le rejet des gaz expirés lorsque le ranimateur comporte un dispositif "déverseur", qui permet l'expiration des gaz à la pression atmosphérique. En l'absence de pression sur la face 25a du piston, la sortie 18c est fermée par le ressort 26 agissant sur la face 25b du piston. Quand une pression est exercée sur la face 25a, le piston se déplace contre le ressort 26 et dégage la sortie 18c.

Un exemple de cellule logique est représenté schématiquement sur la figure 2. Une cellule pneumatique logique est un dispositif pneumatique alimenté en continu par du gaz sous pression et fournissant en sortie un signal pneumatique discontinu, tout ou rien défini par la fréquence des périodes et le rapport temps de signal/temps d'absence de signal. Ces dispositifs de type connu ou résultant de l'association de composants pneumatiques connus, sont essentiellement formés de valves bi-stables associées à des résistances et capacités pneumatiques suivant les lois régissant les oscillateurs pneumatiques. La cellule représentée est constituée par une boite plate 33 séparée en deux parties 33a et 33b par une membrane 31. L'alimentation en gaz à pression absolue constante est assurée par une conduite 32 débouchant dans la partie 33b par l'entrée 11a de la cellule. Un tube 35, formant une capacité, traverse la paroi extérieure de la partie 33b et s'étend jusqu'à la membrane 31 dont le déplacement, sous l'effet des pressions régnant dans les parties 33a et 33b, et sous l'effet d'un ressort 36, obture ou dégage l'extrémité intérieure du tube 35. Le tube 35 et la partie 33a sont connectés par une tubulure 38 munie d'une restriction 39. La sortie (échappement) 11b de la cellule se trouve sur la partie extérieure du tube 35. Une telle cellule est bien connue et il n'est pas nécessaire de s'étendre sur son fonctionnement qui résulte d'ailleurs clairement de la structure décrite. Le tarage du ressort et de la restriction 39 permettent de régler les caractéristiques de la cellule (fréquence et durée des périodes d'ouverture).

On a représenté en haut de la figure les moyens d'alimentation en gaz de la cellule et du distributeur.

Une source de gaz sous pression 40 fournit du gaz respirable et est alimentée à partir de l'extérieur de la chambre 10. Cette source débite dans une canalisation 41 desservant d'un côté un dispositif 12 d'alimentation en gaz à une pression de valeur absolue constante, et de l'autre un dispositif 19 de gaz respirable sous une pression proportionnelle à la pression régnant dans la chambre.

Le dispositif 12 comporte un détendeur constitué de façon à fermer le passage entre son entrée 12a et sa sortie 12b, sur laquelle est branchée l'alimentation 32 de la cellule 11, quand la pression dans la chambre de passage 45 est supérieure à la pression absolue choisie pour l'alimentation de la cellule. Dans le dôme 46, séparé par la membrane 47, est logé un ressort dont le tarage détermine la pression. Ce dôme 46 est étanche et est directement raccordé par une conduite 49 au collecteur 30 débouchant à l'atmosphère. Le fonctionnement d'un tel détendeur est bien connu.

Le dispositif 19 est aussi du type détendeur, avec une chambre de passage 51, une entrée 19a et une sortie 19b, alimentant le distributeur 18 par une canalisation 52. Mais, il comporte une double membrane différentielle 54, 55 délimitant une chambre inerte 57 et un dôme 58. Les deux membranes 54,55 sont connectées par l'entretoise rigide du clapet et ont des surfaces différentes. La chambre 57 qu'elles définissent entre elles est reliée au collecteur 30 à la pression atmosphérique de référence par une canalisation 60. Le dôme 58 renferme un ressort de pression 61 et est en communication avec la chambre hyperbare.

Un tel dispositif 19 fournit un débit d'insufflation à une pression qui est proportionnelle à la pression de la chambre à une constante près, le rapport des pressions étant égal au rapport des surfaces. Si l'on appelle Pu la pression d'utilisation, Pa la pression atmosphérique et Pc la pression de la chambre, R la force du ressort à l'équilibre et S et s les surfaces des membranes, on a :

$$Pu \ s + Pa \ (S - s) = Pc \ S + R$$

soit

$$Pu = Pc \ \frac{S}{s} + \frac{R - Pa \ (S - s)}{s}$$

C'est donc le rapport des surfaces qui fixe la proportionnalité des pressions : pression d'insufflation par rapport à la pression de la chambre. La tension du ressort agissant sur l'ensemble des membranes définit la constante.

On a ainsi une cellule dont l'alimentation est indépendante de la pression dans la chambre, et dont le fonctionnement ne sera donc pas perturbé par cette pression, tandis que la pression d'alimentation du patient sera proportionnelle à la pression de la chambre, afin d'obtenir un débit volumétrique constant, ce qui est le but recherché.

Il va de soi que le mode de réalisation décrit n'est qu'un exemple et qu'il serait possible de le modifier, notamment par substitution d'équivalents techniques, sans sortir pour cela du cadre de l'invention.

- 8 -

Revendications

1. Appareil respiratoire pneumatique volumétrique pour la réanimation en chambre hyperbare, caractérisé en ce qu'il comporte pour la détermination de la fréquence et du rapport I/E de l'inspiration à l'expiration une cellule pneumatique logique (11), un organe (12) d'alimentation de gaz de commande de la cellule à pression de valeur absolue constante, l'échappement (11b) de la cellule étant renvoyé à l'extérieur de la chambre hyperbare, le conduit d'échappement (13) comportant une résistance de charge (14), et une dérivation (16), branchée entre l'échappement de la cellule et la résistance et connectée à un organe de commutation (18) sensible à la pression, pour commander le passage du gaz respirable.

2. Appareil selon la revendication 1,caractérisé en ce qu'il comporte une source de gaz respirable dont la pression est fournie sous une pression relative proportionnelle à celle qui règne dans le caisson, la conduite (21) d'insufflation de gaz comportant un robinet (22) de réglage de passage.

3. Appareil selon la revendication 1, caractérisé en ce que l'organe (18) de commutation sensible à la pression est un distributeur à tiroir comportant un piston (25) ayant deux faces égales, l'espace formé devant une première face (25a) du piston étant en communication avec la sortie (11b) de la cellule, et l'espace formé devant une seconde face (25b) du piston étant connecté à l'extérieur de la chambre hyperbare, cette deuxième face étant chargée par un ressort (26), le distributeur étant par ailleurs connecté à la source de gaz respirable (19) et ayant une conduite d'insufflation de gaz.

4. Appareil selon une des revendications précédentes, caractérisé en ce que l'organe d'alimentation (12) de la cellule logique est un détendeur dont le dôme (46) comportant le ressort de réglage (48) est étanche et directement relié à la pression atmosphérique extérieure par un conduit pneumatique (49).

5. Appareil selon une des revendications 2 à 4, caractérisé en ce que l'organe (19) d'alimentation du distributeur est un détendeur à double membrane différentielle, l'intervalle entre les deux membranes (54,55) étant maintenu sous la pression de référence atmosphérique par un conduit pneumatique (60) de liaison à la pression extérieure à la chambre.

6. Appareil selon une des revendications précédentes, caractérisé en ce que les connexions vers l'extérieur de la chambre hyperbare sont branchées sur un collecteur commun (30) débouchant à l'extérieur de la chambre hyperbare.

*Fig. 1*

Fig. 2

0107590

0107590

Numero de la demande

EP 83 40 2035

**Office européen des brevets**

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | US-A-3 820 566 (SUNDBLOM et al.) <br> * abr g; figures; colonne 4, ligne 28 - colonne 5, ligne 33 * | 1 | A 61 M 16/00 |
| | --- | | |
| A | US-A-3 991 790 (G.K. RUSSELL) <br><br> * abr g; figures; colonne 1, lignes 20-50 * | | |
| | --- | | |
| A | GB-A-1 357 635 (MERICOR MUVEK) | | |
| | ----- | | |

**DOMAINES TECHNIQUES RECHERCHES (Int. Cl. 3)**

A 61 M
A 61 H
B 63 C

Le présent rapport de recherche a été établi pour toutes les revendications

| Lieu de la recherche <br> LA HAYE | Date d'achèvement de la recherche <br> 14-12-1983 | Examinateur <br> FERGUSON J.R. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82